# EUROPEAN PATENT APPLICATION

(11) **EP 2 295 048 A1**
(43) Date of publication of application: **16.03.2011**
(21) Application number: 10011254.9
(22) Date of filing: 26.02.2005
(51) Int. Cl.: A61K 31/12, A61K 31/557, A61P 29/00, A61K 45/06, A61K 36/185

(54) **Synergistic anti-inflammatory pharmaceutical compositions comprising IAA and RIAA**

(30) Priority: 27.02.2004 US 789814
(62) Divisional of application: 05723895.8
(71) Applicant: Metaproteomics, LLC, San Clemente, CA 92673 (US)
(72) Inventor: Babish, John G., Brooktondale, NY 14817 (US); Tripp, Matthew L., Gig Harbour, WA 98335 (US); Bland, Jeffrey S., Fox Island, WA 98333 (US)
(74) Representative: Müller-Boré & Partner Patentanwälte

(57) **Abstract**

The present invention relates to compositions containing a reduces isoalpha acid and an isoalpha acid for use in reducing PGE2-mediated inflammation.

## Description

### Background of the Invention

This invention relates to pharmaceutical compositions containing hops (Humulus lupulus) extracts or derivatives thereof. The present invention also relates to methods of using compositions containing fractions isolated or derived from hops to reduce inflammation.

Prostaglandins (PGs) are ubiquitous hormones that function as both paracrine and autocrine mediators to affect a myriad of physiological changes in the immediate cellular environment. The varied physiological effects of PGs include inflammatory reactions such as rheumatoid arthritis and osteoarthritis, blood pressure control, platelet aggregation, induction of labor and aggravation of pain and fever. The discovery 30 years ago that aspirin and other non-steroidal analgesics inhibited PG production identified PG synthesis as a target for drug development. There are at least 16 different PGs in nine different chemical classes, designated PGA to PGI. PGs are part of a larger family of 20-carbon-containing compounds called eicosanoids; they include prostacyclins, thromboxanes, and leukotrienes. The array of PGs produced varies depending on the downstream enzymatic machinery present in a particular cell type. For example, endothelial cells produce primarily PGI₂, whereas platelets mainly produce TXA₂.

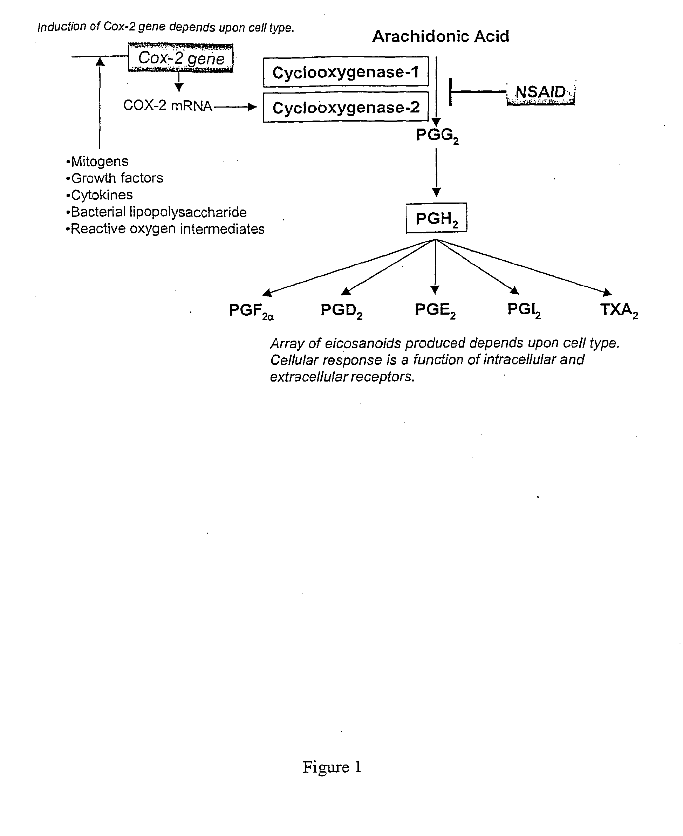

Arachidonic acid serves as the primary substrate for the biosynthesis of all PGs. Cyclooxygenase (prostaglandin endoperoxide synthase, EC 1.14.991, COX) catalyzes the rate-limiting step in the metabolism of arachidonic acid to prostaglandin H₂ (PGH₂), which is further metabolized to various prostaglandins, prostacyclin and thromboxane A2 (see Figure 1). In the early 1990s, it was established that COX exists in two isoforms, commonly referred to as COX-1 and COX-2. It was subsequently determined that the COX-1 and COX-2 proteins are derived from distinct genes that diverged well before birds and mammals. PGs generated via the COX-1 and COX-2 pathways are identical molecules and therefore have identical biological effects. COX-1 and COX-2, however, may generate a unique pattern and variable amounts of eicosanoids; therefore, relative differences in the activation of these isozymes may result in quite dissimilar biological responses. Differences in the tissue distribution and regulation of COX-1 and COX-2 are now considered crucial for the beneficial as well as adverse effects of COX inhibitors.

The generally held concept (COX dogma) is that COX-1 is expressed constitutively in most tissues whereas COX-2 is the inducible enzyme triggered by pro-inflammatory stimuli including mitogens, cytokines and bacterial lipopolysaccharide (LPS) in cells *in vitro* and in inflamed sites *in vivo*. Based primarily on such differences in expression, COX-1 has been characterized as a housekeeping enzyme and is thought to be involved in maintaining physiological functions such as cytoprotection of the gastric mucosa, regulation of renal blood flow, and control of platelet aggregation. COX-2 is considered to mainly mediate inflammation, although constitutive expression is found in brain, kidney and the gastrointestinal tract.

Prostaglandins (PG) are believed to play an important role in maintenance of human gastric mucosal homeostasis. Current dogma is that COX-1 is responsible for PG synthesis in normal gastric mucosa in order to maintain mucosal homeostasis and that COX-2 is expressed by normal gastric mucosa at low levels, with induction of expression during ulcer healing, following endotoxin exposure or cytokine stimulation. It now appears that both COX-1 and COX-2 have important physiological roles in the normal gastric mucosa.

Compounds that inhibit the production of PGs by COX have become important drugs in the control of pain and inflammation. Collectively these agents are known as non-steroidal anti-inflammatory drugs (NSAIDs) with their main indications being osteoarthritis and rheumatoid arthritis. However, the use of NSAIDs, and in particular aspirin, has been extended to prophylaxis of cardiovascular disease. Over the last decade, considerable effort has been devoted to developing new molecules that are direct inhibitors of the enzymatic activity of COX-2, with the inference that these compounds would be less irritating to the stomach with chronic use.

The major problem associated with ascertaining COX-2 selectivity (i.e. low gastric irritancy) is that differences in assay methodology can have profound effects on the results obtained. Depicted in Table 1 are the categories of the numerous *in vitro* assays that have been developed for testing and comparing the relative inhibitory activities of NSAID and natural compounds against COX-1 and COX-2. These test systems can be classified into three groups: (1) systems using animal enzymes, animal cells or cell lines, (2) assays using human cell lines, or human platelets and monocytes, and (3) currently evolving models using human cells that are representative of the target cells for the anti-inflammatory and adverse effects of NSAID and dietary supplements. Generally, models using human cell lines or human platelets and monocytes are the current standard and validated target cell models have not been forthcoming. A human gastric cell line capable of assessing potential for gastric irritancy is a critical need.

The enzymes used can be of animal or human origin, they can be native or recombinant, and they can be used either as purified enzymes, in microsomal preparations, or in whole-cell assays. Other system variables include the source of arachidonic acid. PG synthesis can be measured from endogenously released arachidonic acid or exogenously added arachidonic acid. In the later case, different concentrations are used in different laboratories.

An ideal assay for COX-2 selectivity would have the following characteristics: (1) whole cells should be used that contain native human enzymes under normal physiological control regarding expression; (2) the cells should also be target cells for the anti-inflammatory and adverse effects of the compounds; (3) COX-2 should be induced, thereby simulating an inflammatory process, rather than being constitutively expressed; and (4) PG synthesis should be measured from arachidonic acid released from endogenous stores rather than from exogenously added arachidonic acid.

**Table 1. Classification of test systems for in vitro assays assessing COX-2 selectivity of anti-inflammatory compounds†**

| **I. EST SYSTEMS** | | |
|---|---|---|
| A. ANIMAL | B. HUMAN | C. TARGET |
| Enzymes | Enzymes | Human Gastric Mucosa Cells |
| Cells | Cells | Human Chondrocytes |
| Cell lines | Cell lines | Human Synoviocytes |
| | | |
| D. OTHER SYSTEM VARIABLES | | |
| 1. Source of arachidonic acid - endogenous or exogenous; | | |
| 2. Various expression systems for gene replication of COX-1 and COX-2; | | |
| 3. The presence or absence of a COX-2 inducing agent; | | |
| 4. COX-2 inducing agents are administered at different concentrations and for different periods of time; | | |
| 5. Duration of incubation with the drug or with arachidonic acid; | | |
| 6. Variation in the protein concentration in the medium. | | |

| | | |
|---|---|---|
| †Adapted from Pairet, M. and van Ryn, J. (1993) Experimental models used to investigate the differential inhibition of cyclooxygenase-1 and cyclooxygenase-2 by non-steroidal anti-inflammatory drugs. Inflamm. Res 47, Supplement 2S93-S101 and incorporated herein by reference. | | |

No laboratory has yet developed an ideal assay for COX-2 selectivity. The whole cell system most commonly used for prescription (Rx) and over the counter (OTC) products is the human whole blood assay developed by the William Harvey Institute (Warner et al., Proc Natal Acad Sci U S A 96:7563-7568(1999)). To date, this assay format has developed more data supporting clinical relevance than any other. However, new research on the role of constitutive expression of COX-2 in normal gastric mucosa necessitates revisiting the relevance of the use of platelets to model COX-1 inhibition in the absence of COX-2. The extrapolation of gastrotoxicity from platelet studies is no longer on a sound molecular basis. The validation of a human gastric mucosal cell line for establishing the potential target tissue toxicity of cyclooxygenase inhibitors represents a critical need for the development of safe and effective anti-inflammatory agents.

An ideal formulation for the treatment of inflammation would inhibit the induction and activity of COX-2 without inhibiting the synthesis of PGE₂ in gastric mucosal cells. However, conventional non-steroidal anti-inflammatory drugs lack the specificity of inhibiting COX-2 without affecting gastric PGE₂ synthesis and are at risk to cause damages on the gastrointestinal system, when used for extended periods. Indeed, even the newly developed, anti-inflammatory drugs such as rofecoxib (Vioxx®, Merck & Co., Inc.) and celecoxib (Celebrex®, Pfizer, Inc.) produce untoward gastric toxicity in the form of induced spontaneous bleeding and delay of gastric ulcer healing.

### NSAID Toxicity

NSAIDs are known to cause serious health problems including gastric bleeding and kidney damage. In the United States, there are over 13 million regular users of NSAIDs, 70 million NSAID prescriptions written every year, and 30 billion over the counter NSAIDs tablets sold annually. NSAID-induced disease causes 103,000 hospitalizations per year and an estimated 16,500 deaths annually. Twenty percent of all chronic NSAID users will develop a peptic ulcer. NSAID users have a greater risk - three to four times higher - to upper gastrointestinal bleeding, perforation, or both. Eighty-one percent of patients hospitalized with serious NSAID-induced complications had no previous gastrointestinal symptoms. People over 60 years of age have a significantly higher probability of experiencing complications associated with NSAID use. Moreover, 21% of all adverse drug reaction in the United States are due to NSAID use.

The new selective COX-2 inhibitors such as celecoxib and rofecoxib have been shown to offer a safer alternative to most NSAIDs. However recent studies indicate that selective COX-2 inhibitors do not completely eliminate gastrointestinal toxicity. In fact in cases of inflammation or ulceration of the gastrointestinal tract, prescription COX-2 inhibitors may delay ulcer healing.

Thus, it would be useful to identify a natural formulation of compounds that would specifically inhibit or prevent the synthesis of prostaglandins by COX-2 with little or no effect on synthesis of PGE₂ in the gastric mucosa. Such a formulation would be useful for preserving the health of joint tissues, for treating arthritis or other inflammatory conditions. The term "specific or selective COX-2 inhibitor" was coined to embrace compounds or mixtures of compounds that selectively inhibit COX-2 over COX-1. However, while the implication is that such a calculated selectivity will result in lower gastric irritancy, unless the test materials are evaluated in gastric cells, the term "selective COX-2 inhibitor" does not carry assurance of safety to gastrointestinal cells. Only testing of compound action in target tissues, inflammatory cells and gastric mucosal cells will identify those agents with low potential for stomach irritation.

Therefore, it would be useful to identify a composition that would specifically inhibit or prevent the expression of COX-2 enzymatic activity in inflammatory cells, while having little or no effect on PGE₂ synthesis in gastric mucosal cells so that these formulations could be used with no gastrointestinal upset. Furthermore, such formulations should allow for healing of pre-existing ulcerative conditions in the stomach. The present invention satisfies this need and provides related advantages as well.

### Summary of the Invention

The invention provides a composition comprising a reduced isoalpha acid (RIAA) and isoalpha acid (IAA) isolated from hops, wherein the RIAA and IAA are in a ratio of about 3:1 to about 1:10. The invention also provides a method of reducing inflammation by administering a composition comprising a reduced isoalpha acid (RIAA) and isoalpha acid (IAA) isolated from hops, wherein the RIAA and IAA are in a ratio of about 3:1 to about 1:10.

### Brief Description of the Drawings

Figure 1 depicts the induction of cyclooxygenase-2 and the metabolism of arachidonic acid to prostaglandins and other eicosanoids by the cyclooxygenase enzymes. The action of non-steroidal anti-inflammatory agents is through direct inhibition of the cyclooxygenase enzymes.

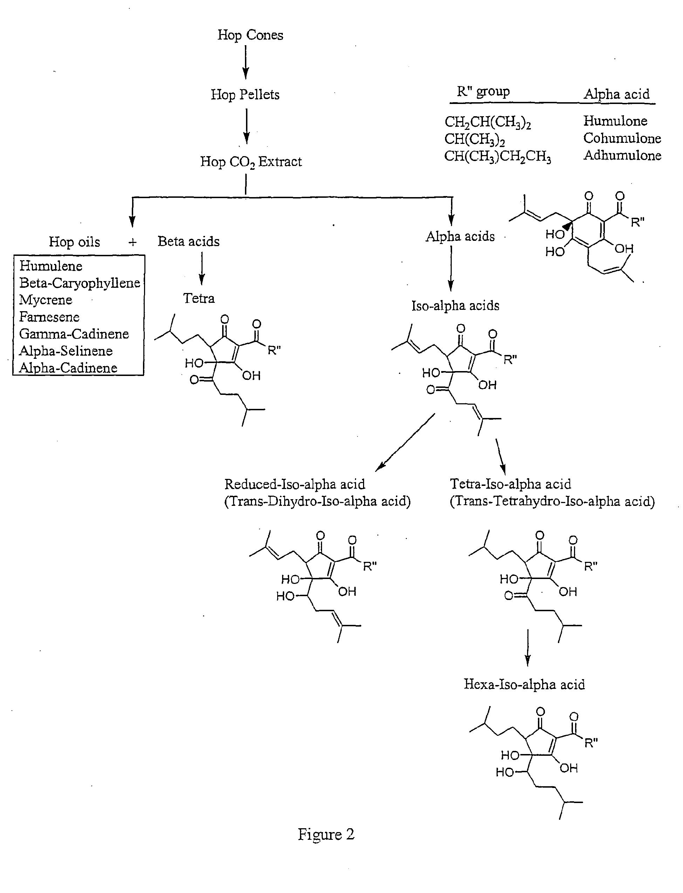

Figure 2 shows an outline of fractions and compounds that can be obtained from hops.

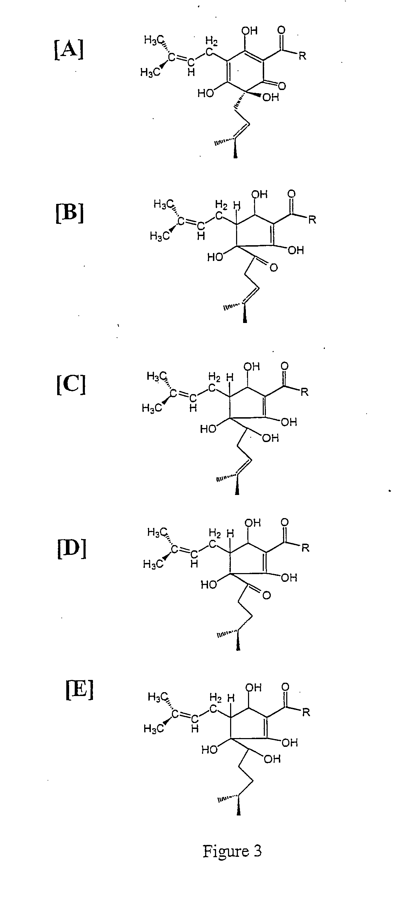

Figure 3 illustrates exemplary fractions isolated or derived from hops. Figure 3A shows the alpha-acid genus (AA) and representative species humulone (R= - CH₂CH(CH₃)₂), cohumulone (R=, -CH(CH₃)₂), and adhumulone (R= - CH(CH₃)CH₂CH₃); Figure 3B shows the isoalpha acid genus (IAA) and representative species isohumulone (R= -CH₂CH(CH₃)₂), isocohumulone (R=, -CH(CH₃)₂), and isoadhumulone (R= -CH(CH₃)CH₂CH₃); Figure 3C shows the reduced isomerized isoalpha acid genus (RIAA) and representative species dihydro-isohumulone (R= - CH₂CH(CH₃)₂) dihydro-isocohumulone (R=, -CH(CH₃)₂), and dihydro-adhumulone (R= - CH(CH₃)CH₂CH₃); Figure 3D shows the tetra-hydroisoalpha acid genus (THIAA) and representative species tetra-hydro-isohumulone (R= -CH₂CH(CH₃)₂), tetra-hydro-isocohumulone ((R=, -CH(CH₃)₂), and tetra-hydro-adhumulone (R= -CH(CH₃)CH₂CH₃); Figure 3E shows and the hexa-hydroisoalpha acid (HHIAA) genus with representative species hexa-hydro-isohumulone (R= -CH₂CH(CH₃)₂) hexa-hydro-isocohumulone (R=, - CH(CH₃)₂), and hexa-hydro-adhumulone (R= -CH(CH₃)CH₂CH₃).

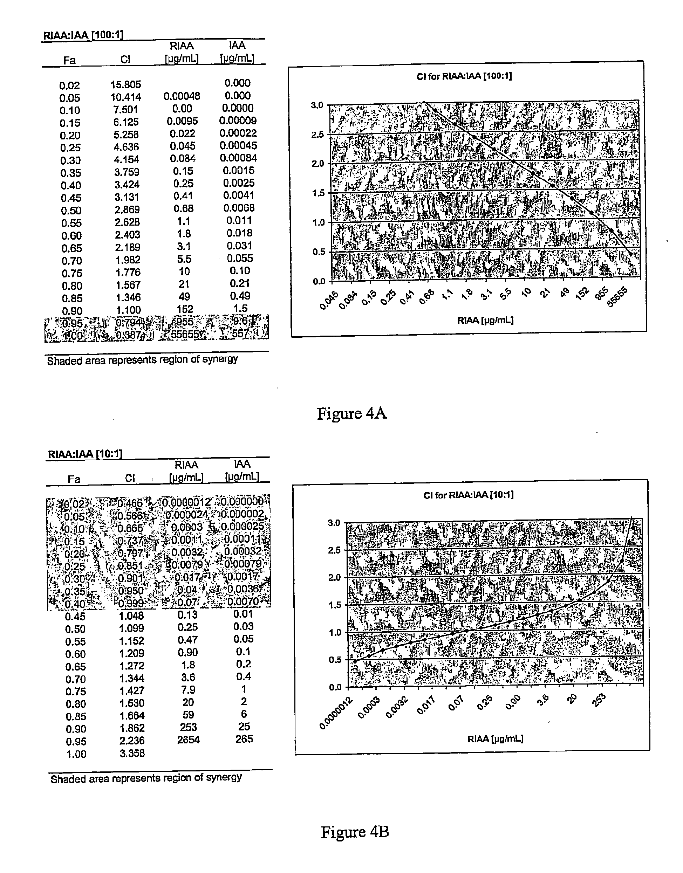

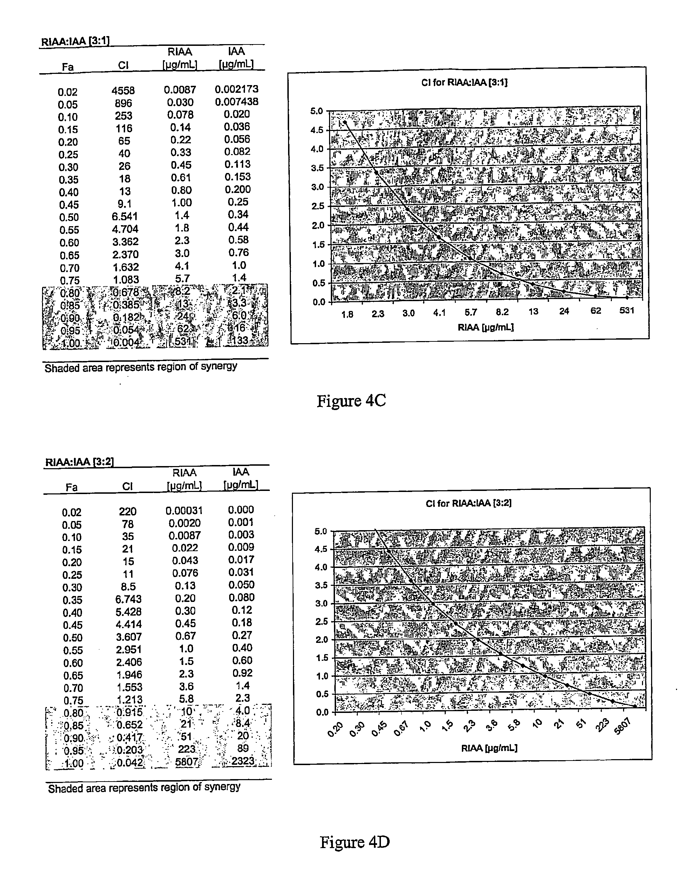

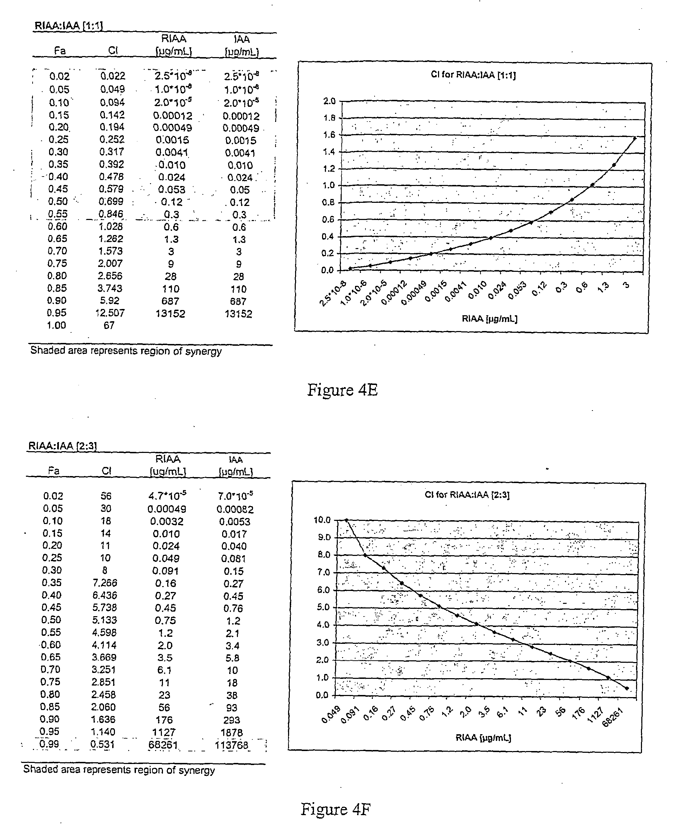

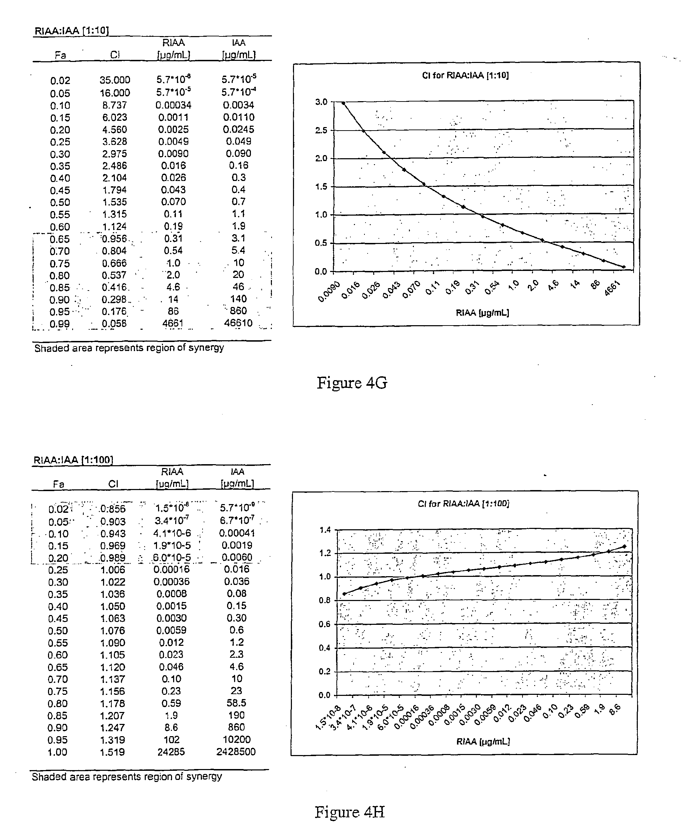

Figure 4 shows a graphic representation of the computed Combination Index parameter versus the concentration of reduced isomerized alpha-acids (RIAA), isomerized alpha-acids (IAA), and for RIAA:IAA ratios of 100:1 (Figure 4A), 10:1 (Figure 4B), 3:1 (Figure 4C), 3:2, (Figure 4D), 1:1 (Figure 4E), 2:3 (Figure 4F), 1:10 (Figure 4G), 1:100 (Figure 4H).

### Detailed Description of the Invention

The present invention provides compositions and methods for reducing inflammation. In particular, the invention provides components isolated or derived from hops (*Humulus lupulus*) that synergistically inhibit prostaglandin E₂ (PGE₂). Such compositions can be used in methods to reduce inflammation. The invention provides hops (*Humulus lupulus*) extracts or derivatives thereof for use in treating inflammation in a patient prophylactically and/or therapeutically. For example, the invention provides a combination of one or more fractions isolated or derived from hops that synergistically inhibit inflammation. In a particular embodiment, the invention provides methods of reducing inflammation by administering a combination of an isoalpha acid and reduced isoalpha acid from hops, which synergistically inhibits PGE₂.

The acute toxicity of hops derivatives is very low. Therefore, relatively high doses of hops derivatives can be used, if desired, without toxic effects due to the hops. Toxic doses are considerably higher than the therapeutic doses contemplated in accordance with the present invention.

As used herein, the term "dietary supplement" refers to compositions consumed to affect structural or functional changes in physiology. The term "therapeutic composition" refers to compounds administered to treat or prevent a disease or to ameliorate a sign or symptom associated with a disease.

As used herein, the term "effective amount" means an amount necessary to achieve a selected result. Such an amount can be readily determined without undue experimentation by a person of ordinary skill in the art.

As used herein, the term "substantial" means being largely but not wholly that which is specified.

As used herein, the term "COX inhibitor" refers to a composition of compounds that is capable of inhibiting the activity or expression of COX-2 enzymes or is capable of inhibiting or reducing the severity, including pain and swelling, of a severe inflammatory response.

As used herein, the terms "derivatives" or a matter "derived" refer to a chemical substance related structurally to another substance and theoretically obtainable from it, that is, a substance that can be made from another substance. Derivatives can include compounds obtained via a chemical reaction. Methods of making derivatives of compounds are well known to those skilled in the art.

As used herein, the term "inflammatory cell" refers to those cellular members of the immune system, for example B and T lymphocytes, neutrophils or macrophages, involved in synthesis of prostaglandins in response to inflammatory signals such as interleukins, tumor necrosis factor, bradykinin, histamine or bacterial-derived components.

As used herein, the term "target cells" refers to that cell population in which the inhibition of PGE₂ or other prostaglandin synthesis is desired, such as inflammatory cells or tumor cells. Alternatively, "non-target cells" refers to that cell population in which the inhibition of PGE₂ or other prostaglandin synthesis is not desired, such as the gastric mucosal, neural or renal cells.

As used herein, the term "hop extract" refers to the solid material resulting from (1) exposing a hops plant product to a solvent, (2) separating the solvent from the hops plant products, and (3) eliminating the solvent.

As used herein, the term "solvent" refers to a liquid of aqueous or organic nature possessing the necessary characteristics to extract solid material from the hop plant product. Examples of solvents would include, but are not limited to, water, steam, superheated water, methanol, ethanol, hexane, chloroform, methylene chloride, liquid or supercritical CO₂, liquid N₂, or combinations of such materials.

As used herein, the term "CO₂ extract" refers to the solid material resulting from exposing a hops plant product to a liquid or supercritical CO₂ preparation followed by the removing of the CO₂.

As used herein, the term "spent hops" refers to the solid and hydrophilic residue from extract of hops.

As used herein, the term "alpha acid" refers to compounds collectively known as humulones and can be isolated from hops plant products including, among others, humulone, cohumulone, adhumulone, hulupone, and isoprehumulone.

As used herein, the term "isoalpha acid" refers to compounds isolated from hops plant products and which subsequently have been isomerized. The isomerization of alpha acids can occur thermally, such as boiling. Examples of isoalpha acids include, but are not limited to, isohumulone, isocohumulone, and isoadhumulone.

As used herein, the term "reduced isoalpha acid" refers to alpha acids isolated from hops plant product and which subsequently have been isomerized and reduced, including cis and trans forms. Examples of reduced isoalpha acids (RIAA) include, but are not limited to, dihydro-isohumulone, dihydro-isocohumulone, and dihydro-adhumulone.

As used herein, the term "tetra-hydroisoalpha acid" refers to a certain class of reduced isoalpha acid. Examples of tetra-hydroisoalpha acid (THIAA) include, but are not limited to, tetra-hydro-isohumulone, tetra-hydro-isocohumulone and tetra-hydro-adhumulone.

As used herein, the term "hexa-hydroisoalpha acid" refers to a certain class of reduced isoalpha acid. Examples of hexa-hydroisoalpha acids (HHIAA) include, but are not limited to, hexa-hydro-isohumulone, hexa-hydro-isocohumulone and hexa-hydro-adhumulone.

As used herein, the term "beta-acid fraction" refers to compounds collectively known as lupulones including, among others, lupulone, colupulone, adlupulone, tetrahydroisohumulone, and hexahydrocolupulone.

As used herein, the term "essential oil fraction" refers to a complex mixture of components including, among others, myrcene, humulene, beta-caryophyllene, undecane-2-on, and 2-methyl-but-3-en-ol.

As used herein, "conjugates" of compounds means compounds covalently bound or conjugated to a member selected from the group consisting of mono- or disaccharides, amino acids, sulfates, succinate, acetate, and glutathione. The mono- or disaccharide can be a member selected from the group consisting of glucose, mannose, ribose, galactose, rhamnose, arabinose, maltose, and fructose.

The invention relates to using hops extracts to reduce inflammation. Hop extraction in one form or another goes back over 150 years to the early nineteenth century when extraction in water and ethanol was first attempted. Even today, an ethanol extract is available in Europe, but by far the predominant extracts are organic solvent extracts (for example, hexane) and CO₂ extracts (supercritical and liquid). CO₂ (typically at 60 bars pressure and 50 to 10°C) is in a liquid state and is a relatively mild, non-polar solvent highly specific for hop soft resins and oils. Beyond the critical point, typically at 300 bars pressure and 60°C, CO₂ has the properties of both a gas and a liquid and is a much stronger solvent. The composition of the various extracts is compared in Table 2.

At its simplest, hop extraction involves milling, pelleting and re-milling the hops to spread the lupulin, passing a solvent through a packed column to collect the resin components and finally, removal of the solvent to yield a whole or "pure" resin extract.

**Table 2. Hop extracts (Percent w/w)**

| Component | Hops | Organic Solvent | Super-Critical CO₂ | Liquid CO₂ |
|---|---|---|---|---|
| Total resins | 12-20 | 15-60 | 75-90 | 70-95 |
| Alpha-acids | 2-12 | 8-45 | 27-55 | 30-60 |
| Beta-acids | 2-10 | 8-20 | 23-33 | 15-45 |
| Essential oils | 0.5-1.5 | 0-5 | 1-5 | 2-10 |
| Hard resins | 2-4 | 2-10 | 5-11 | None |
| Tannins | 4-10 | 0.5-5 | 0.1-5 | None |
| Waxes | 1-5 | 1-20 | 4-13 | 0-10 |
| Water | 8-12 | 1-15 | 1-7 | 1-5 |

The main organic extractants are strong solvents and in addition to virtually all the lupulin components, they extract plant pigments, cuticular waxes, water and water-soluble materials.

Supercritical CO₂ is more selective than the organic solvents and extracts less of the tannins and waxes and less water and hence water-soluble components. It does extract some of the plant pigments like chlorophyll but rather less than the organic solvents do. Liquid CO₂ is the most selective solvent used commercially for hops and hence produces the most pure whole resin and oil extract. It extracts hardly the hard resins or tannins, much lower levels of plant waxes, no plant pigments and less water and water-soluble materials.

As a consequence of this selectivity and the milder solvent properties, the absolute yield of liquid CO₂ extract per unit weight of hops is less than when using the other mentioned solvents. Additionally, the yield of alpha acids with liquid CO₂ (89-93%) is lower than that of supercritical CO₂ (91-94%) or the organic solvents (93-96%). Following extraction, there is the process of solvent removal, which for organic solvents involves heating to cause volatilization. Despite this, trace amounts of solvent do remain in the extract. The removal of CO₂, however, simply involves a release of pressure to volatize the CO₂.

As shown in Figure 3, hops CO₂ extracts can be fractionated into components, including hops oils, beta acids, and alpha acids. Hops oils include, but are not limited to, humulene, beta-caryophyllene, mycrene, farnescene, gamma-cadinene, alpha-selinene, and alpha-cadinene. Beta acids include, but are not limited to, lupulone, colupulone, adlupulone, tetrahydroisohumulone, and hexahydrocolupulone, collectively known as lupulones. Beta acids can be isomerized and reduced. Beta acids are reduced to give tetra-beta acids. Alpha acids include, but are not limited to, humulone, cohumulone, adhumulone, hulupone, and isoprehumulone. Alpha acids can be isomerized to give isoalpha acids. Iso-alpha acids can be reduced to give reduced-isoalpha acids, tetra-hydroisoalpha acids, and hexa-hydroisoalpha acids.

The identification of humulone from hops extract as an inhibitor of bone resorption is reported in Tobe et al. (Biosci. Biotech. Biochem 61(1):158-159 (1997)). Later studies by the same group characterized the mechanism of action of humulone as inhibition of COX-2 gene transcription following TNFalpha stimulation of MC3T3, El cells (Yamamoto, FEBS Letters 465:103-106 (2000)). It was concluded that the action of humulone (also humulon) was similar to that of glucocorticoids, but that humulone did not function through the glucocorticoid receptor. While these results establish that humulone inhibits PGE₂ synthesis in MC3T3 cells (osteoblasts) at the gene level, one skilled in the art would not assume that these results would necessarily occur in immune inflammatory cells or other cell lines. As disclosed herein, hops compounds and derivatives exhibit a high degree of tissue selectivity in target and non-target cells. Furthermore, the hops derivatives described in the present invention are structurally distinct from the alpha acid humulone.

The invention provides compositions containing at least one fraction isolated or derived from hops (*Humulus lupulus*). Examples of fractions isolated or derived from hops are alpha acids, isoalpha acids, reduced isoalpha acids, tetra-hydroisoalpha acids, hexa-hydroisoalpha acids, beta acids, and spent hops. Fractions isolated or derived from hops, include, but are not limited to, cohumulone, adhumulone, isohumulone, isocohumulone, isoadhumulone, dihydro-isohumulone, dihydro-isocohumulone, dihydro-adhumulone, tetrahydro-isohumulone, tetrahydro-isocohumulone, tetrahydro-adhumulone, hexahydro-isohumulone, hexahydro-isocohumulone, and hexahydro-adhumulone. Preferred compounds can also bear substituents, such as halogens, ethers, and esters.

Compounds of the fractions isolated or derived from hops can be represented by a supragenus below: 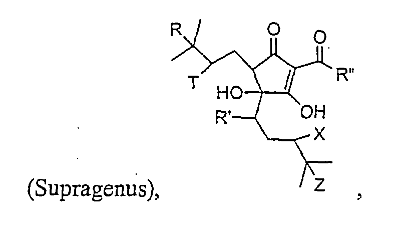 wherein R' is selected from the group consisting of carbonyl, hydroxyl, OR, and OCOR, wherein R is alkyl; wherein R" is selected from the group consisting of CH(CH₃)₂, CH₂CH(CH₃)₂, and CH(CH₃)CH₂CH₃; and wherein R, T, X, and Z are independently selected from the group consisting of H, F, Cl, Br, I, and π orbital, with the proviso that if one of R, T, X, or Z is a π orbital, then the adjacent R, T, X, or Z is also a π orbital, thereby forming a double bond.

In another embodiment, compounds of the fractions isolated or derived from hops can be represented by a genus below: 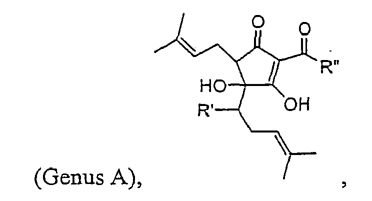 wherein R' is selected from the group consisting of carbonyl, hydroxyl, OR, and OCOR, wherein R is alkyl; and wherein R" is selected from the group consisting of CH(CH₃)₂, CH₂CH(CH₃)₂, and CH(CH₃)CH₂CH₃. Exemplary Genus A structures include isoalpha acids such as isohumulone, isocohumulone, isoadhumulone, and the like, and reduced isoalpha acids such as dihydro-isohumulone, dihydro-isocohumulone, dihydroadhumulone, and ether or ester conjugates or halogenated modifications of the double bond.

In yet another embodiment, compounds of the fractions isolated or derived from hops can be represented by a genus below: 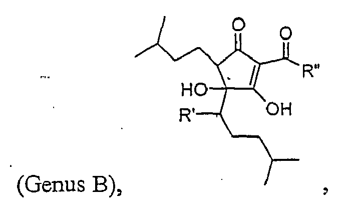 wherein R' is selected from the group consisting of carbonyl, hydroxyl, OR, and OCOR, wherein R is alkyl; and wherein R" is selected from the group consisting of CH(CH₃)₂, CH₂CH(CH₃)₂, and CH(CH₃)CH₂CH₃. Exemplary Genus B structures include tetra-hydroisoalpha acids such as tetra-hydro-isohumulone, tetra-hydro-isocohymulone and tetra-hydro-adhumulone, and the like, and hexa-hydroisoalpha acids such as hexa-hydro-isohumulone, hexa-hydro-isocohumulone and hexa-hydro-adhumulone, and ether or ester conjugates.

As shown in Figure 3, examples of compounds of an ingredient isolated or derived from hops, include, but are not limited to, humulone, cohumulone, adhumulone, isohumulone, isocohumulone, isoadhumulone, dihydro-isohumulone, dihydro-isocohumulone, dihydro-adhumulone, tetrahydro-isohumulone, tetrahydro-isocohumulone, tetrahydro-adhumulone, hexahydro-isohumulone, hexahydro-isocohumulone, and hexahydro-adhumulone. The compounds can bear substituents, as shown in the formula above.

Hops derivatives are known compounds occurring naturally in plants and found in food products and beverages. They may be prepared by any of the extraction and processing methods known in the art. Hops derivatives can be prepared directly from plant material in any known manner. The hops derivatives may be purified by methods known in the art, for example, by recrystallization from aqueous organic solvents such as aqueous alcohols. Synthetic modifications of hops derivatives may be prepared according to methods known in the pharmaceutical art of drug modification.

Also in accordance with the present invention there are provided pharmaceutical compositions comprising an effective amount of hops derivatives optionally in combination with a pharmaceutical diluent or adjuvant.

### Dosage

Further in accordance with the present invention there are provided pharmaceutical formulations of oral dosage forms comprising an effective amount of hops derivatives for release of the active ingredient at a desired site in the gastro-intestinal tract, for instance either in the stomach and/or duodenum according to known formulation techniques, for example, slow releasing tablets. Still further in accordance with the invention, there are provided pharmaceutical compositions comprising an effective tolerated amount of hops derivatives. Due to its low toxicity, high dosages of hops derivatives can be employed to produce useful results, depending upon the particular effect that is desired.

Hops derivatives are particularly suitable for oral administration. Therefore, hops derivatives can be formulated for oral use, namely: tablets, coated tablets, dragees, capsules, powders, granulates and soluble tablets, and liquid forms, for example, suspensions, dispersions or solutions, optionally together with an additional active ingredient.

The invention extends to a method of preparing such pharmaceutical compositions as described herein and compositions when so prepared. The compositions may be manufactured by a method which comprises mixing hops derivatives with a pharmaceutically acceptable carrier or auxiliary, and optionally with an analgesic and/or anti-inflammatory substance and/or another compound(s). Methods for preparing a pharmaceutical composition are well known to those skilled in the art (see, for example, Genarro, ed., Remington's Pharmaceutical Sciences, 18th ed., Mack Publishing Co., Easton, Pennsylvania (1990)).

The selected dosage level will depend upon the activity of the particular composition, the route of administration, the severity of the condition being treated or prevented, and the condition and prior medical history of the patient being treated. However, it is within the skill of the art to start doses of the composition at levels lower than required to achieve the desired therapeutic effect and to gradually increase the dosage until the desired effect is achieved. If desired, the effective daily dose may be divided into multiple doses for purposes of administration, for example, two to four separate doses per day. It will be understood, however, that the specific dose level for any particular patient will depend upon a variety of factors including body weight, general health, diet, time and route of administration, combination with other compositions and the severity of the particular condition being treated or prevented.

The invention provides methods that include delivering an effective amount of hops fractions, hops compounds, or hops derivatives alone or in combination with an additional active ingredient, as disclosed herein. For example, a daily dose of compositions of the invention can be formulated to deliver about 0.5 to about 10,000 mg of a hops fraction, for example, alpha acid, isoalpha acid, reduced isoalpha acid, tetra-hydroisoalpha acid, hexa-hydroisoalpha acid, beta acid, spent hops, or other hops fractions, per day. In particular, an effective daily dose of compositions can be formulated to deliver about 50 to about 7500 mg of hops fraction, for example, alpha acids, isoalpha acid, reduced isoalpha acid, tetra-hydroisoalpha acid, hexa-hydroisoalpha acid, beta acid, spent hops, or other hops fractions, per day. For example, an effective daily dose of compositions can be formulated to deliver about 100 mg to about 5000 mg, about 200 mg to about 3000 mg, about 300 mg to about 2000 mg, about 500 to about 1000 mg of hops fraction per day. In one embodiment, the effective daily dose is administered once or twice a day. A certain embodiment provides a composition comprising about 0.5 to about 500 mg of isoalpha acid or reduced isoalpha acid, for example, about 50 to about 300 mg or about 100 to about 200 mg of isoalpha acid or reduced isoalpha acid per day. In another embodiment, the invention provides a composition comprising about 10 to about 3000 mg of reduced isoalpha acid, tetra-hydroisoalpha acid, or hexa-hydroisoalpha acid per day, for example, about 50 to about 2000 mg, about 100 to about 1000 mg, about 200 to about 750 mg, or about 250 to about 500 mg of reduced isoalpha acid, tetra-hydroisoalpha acid, or hexa-hydroisoalpha acid per day. Yet another certain embodiment provides a composition comprising about 50 to about 7500 mg of spent hops per day, for example, about 100 to about 6000 mg, about 200 to about 5000 mg, about 300 to about 3000 mg, about 500 to about 2000 mg, or about 1000 to about 1500 mg of spent hops per day.

A composition of embodiments for topical application can contain about 0.001 to about 10 weight percent, for example, about 0.01 to about 5 weight percent, or about 0.1 to about 1 weight percent, of a hops derivative. Such compositions can produce serum concentrations in the range of about 0.0001 to about 10 µM, for example, about 0.001 to about 5 µM, about 0.01 to 1 µM, or about 0.1 to about 0.5 µM of a fraction isolated or derived from hops or conjugate thereof.

In a composition of the invention in which one or more fractions isolated or derived from hops is combined, ratios of the one or more fractions can be varied to optimize a desired effect. For example, as disclosed herein, synergy of PGE₂ inhibition by a combination of RIAA and IAA was observed in RAW 264.7 macrophage cells (see Example 6). Synergy between RIAA and IAA was observed in combinations of 3:1, 3:2, 1:1 and 1:10, respectively. Particularly effective synergy was observed at 1:1 and 1:10 RIAA:IAA ratios. The invention provides a composition containing one or more fractions isolated or derived from hops that synergistically inhibits PGE₂. In one embodiment, the invention provides a combination of RIAA and IAA in an amount and ratio effective to synergistically inhibit PGE₂. The RIAA and IAA is combined in an effective ratio to synergistically inhibit PGE₂, for example, a RIAA:IAA ratio of about 3:1, about 3:2 or about1:1 to 1:10, in particular about 1:1, about 1:2, about 1:3, about 1:4, about 1:5, about 1:6, about 1:7, about 1:8, about 1:9, or about 1:10.

### Formulations

Compositions of the invention can be administered in the form of a dietary supplement or therapeutic composition. The compositions may be administered orally, topically, transdermally, transmucosally, parenterally, and the like, in appropriate dosage units, as desired. Compositions for dietary application may include various additives such as other natural components of intermediary metabolism, vitamins and minerals, as well as inert ingredients such as talc and magnesium stearate that are standard excipients in the manufacture of tablets and capsules. For example, one embodiment comprises active ingredients of compositions of the invention in combination with glucosamine or chondrotin sulfate.

As used herein, "pharmaceutically acceptable carrier" includes solvents, dispersion media, coatings, isotonic and absorption delaying agents, sweeteners and the like, suitable for administration to an individual. These pharmaceutically acceptable carriers may be prepared from a wide range of materials including, but not limited to, diluents, binders and adhesives, lubricants, disintegrants, coloring agents, bulking agents, flavoring agents, sweetening agents and miscellaneous materials such as buffers and absorbents that may be needed in order to prepare a particular therapeutic composition. The use of such media and agents for pharmaceutically active substances is well known in the art. It is understood that formulations contain components that are compatible with the active ingredients. In one embodiment, talc, and magnesium stearate are included in the formulation. Other ingredients known to affect the manufacture of a composition of the invention as a dietary bar or functional food can include flavorings, sugars, amino-sugars, proteins and/or modified starches, as well as fats and oils.

Dietary supplements, lotions or therapeutic compositions of embodiments of the invention can be formulated in any manner known by one of skill in the art. In one embodiment, the composition is formulated into a capsule or tablet using techniques available to one of skill in the art. In capsule or tablet form, the recommended daily dose for an adult human or animal can be contained in one to six capsules or tablets. The compositions can also be formulated in other convenient forms, such as an injectable solution or suspension, a spray solution or suspension, a lotion, gum, lozenge, food or snack item. Food, snack, gum or lozenge items can include any ingestible ingredient, including sweeteners, flavorings, oils, starches, proteins, fruits or fruit extracts, vegetables or vegetable extracts, grains, animal fats or proteins. Thus, compositions of the invention can be formulated into cereals, snack items such as chips, bars, gumdrops, chewable candies or slowly dissolving lozenges. Compositions of the invention can be used for the treatment of inflammation-based diseases, both acute and chronic. Particularly useful formulations of compositions of the invention can reduce the inflammatory response and thereby promote healing of, or prevent further damage to, the affected tissue. A pharmaceutically acceptable carrier can also be used in the compositions and formulations of the invention.

Compositions of the invention can be used, for example, for the treatment of inflammation in a subject, and for treatment of other inflammation-associated disorders, such as an analgesic in the treatment of pain and headaches, or as an antipyretic for the treatment of fever. Compositions of the invention can be used to treat arthritis, including but not limited to rheumatoid arthritis, spondyloathopathies, gouty arthritis, osteoarthritis, systemic lupus erythematosis, and juvenile arthritis.

In one embodiment, the invention provides a composition comprising a reduced isoalpha acid (RIAA) and isoalpha acid (IAA) isolated from hops, wherein the RIAA and IAA are in a ratio of about 3:1 to about 1:10. In such a composition, the isoalpha acid can be selected from isohumulone, isocohumulone, and isoadhumulone. The reduced isoalpha acid can be selected from dihydro-isohumulone, dihydro-isocohumulone, and dihydro-adhumulone.

The invention also provides a method of reducing inflammation by administering a composition comprising a reduced isoalpha acid (RIAA) and isoalpha acid (IAA) isolated from hops, wherein the RIAA and IAA are in a ratio of about 3:1 to about 1:10. The isoalpha acid can be selected from isohumulone, isocohumulone, and isoadhumulone. The reduced isoalpha acid can be selected from dihydro-isohumulone, dihydro-isocohumulone, and dihydro-adhumulone.
The invention additionally provides a method of reducing inflammation by administering at least two compounds of Genus A having the formula: 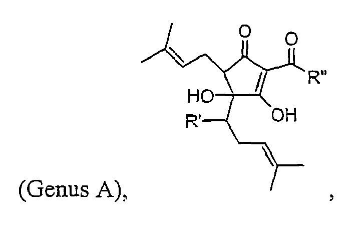 wherein R' is selected from the group consisting of carbonyl, hydroxyl, OR, and OCOR, wherein R is alkyl; and wherein R" is selected from the group consisting of CH(CH₃)₂, CH₂CH(CH₃)₂, and CH(CH₃)CH₂CH₃, wherein the two compounds are in a ratio of about 10:1 to about 1:10.

Besides being useful for human treatment, embodiments of the invention are also useful for treatment of other animals, including horses, dogs, cats, birds, sheep, pigs, and the like. Formulations for the treatment of inflammation can inhibit the induction and activity of COX-2 with little effect on the synthesis of PGE₂ in the gastric mucosa. Historically, the NSAIDs used for treatment of inflammation lacked the specificity of inhibiting COX-2 without affecting PGE₂ synthesis in gastric mucosal cells. Therefore, these drugs irritated and damaged the gastrointestinal system when used for extended periods. Such contraindications are not associated with the present invention and therefore, the formulations described may be used for extended periods with limited or no gastropathy. Administration can be by any method available to the skilled artisan, for example, by oral, topical, transdermal, transmucosal, or parenteral routes.

The invention also provides a method of reducing inflammation by administering an isoalpha acid and reduced isoalpha acid isolated from hops (see Example 6). The combination of isoalpha acid and reduced isoalpha acid synergistically inhibits PGE₂ synthesis in an inflammatory cell model (Example 6).

As used herein, "reducing inflammation" refers to decreasing, ameliorating or inhibiting an inflammatory response. One skilled in the art can readily recognize a reduction in a sign or symptom associated with an inflammatory response. Reducing inflammation can refer to decreasing the severity of a sign or symptom associated with inflammation as well as inhibiting inflammation so that few or no symptoms associated with inflammation are presented.

As disclosed herein, a variety of assays are available to show the effectiveness of one or more fractions isolated or derived from hops (see examples). It is understood by those skilled in the art that a fraction isolated or derived from hops, as disclosed herein, can be assayed for activity in reducing inflammation using a variety of assays well known to those skilled in the art, including those exemplified herein.

The following examples are intended to illustrate but are not intended to limit the scope of the invention.

### EXAMPLE 1

### INHIBITION OF PGE₂ SYNTHESIS IN STIMULATED AND NONSTIMULATED MURINE MACROPHAGES BY HOPS (Humulus lupulus) COMPOUNDS AND DERVIATIVES

Summary - This example illustrates that hops fractions and derivatives inhibit COX-2 synthesis of PGE₂ preferentially over COX-1 synthesis of PGE₂ in the RAW 264.7 murine macrophage model.

*Chemicals and reagents -* Bacterial lipopolysaccharide (LPS; B E. coli 055:B5) was from Sigma (St. Louis, MO). Hops fractions (1) alpha hop (1% alpha acids; AA), (2) aromahop OE (10% beta acids and 2% isomerized alpha acids, (3) isohop (isomerized alpha acids; IAA), (4) beta acid solution (beta acids BA), (5) hexahop gold (hexahydro isomerized alpha acids; HHIAA), (6) redihop (reduced isomerized-alpha acids; RIAA), (7) tetrahop (tetrahydro-iso-alpha acids THIAA) and (8) spent hops were obtained from Betatech Hops Products (Washington, D.C., U.S.A.). The spent hops were extracted two times with equal volumes of absolute ethanol. The ethanol was removed by heating at 40°C until a only thick brown residue remained. This residue was dissolved in DMSO for testing in RAW 264.7 cells. Prostaglandin E2 EIA kit Monoclonal was purchased from Cayman Chemical (Ann Arbor, MI). Anti-COX-1 and anti-COX-2 rabbit polyclonal antisera were obtained from Upstate Biotechnology (Lake Placid, NY); donkey anti-goat IgG-HRP was procured from Santa Cruz Biotechnology (Santa Cruz, CA). Heat inactivated Fetal Bovine Serum (FBS-HI Cat. #35-011CV), and Dulbeco's Modification of Eagle's Medium (DMEM Cat #10-013CV) was purchased from Mediatech (Herndon, VA). Unless otherwise noted, all standard reagents were obtained from Sigma (St. Louis, MO) and were the purest commercially available.

Equipment used in this example included: an OHAS Model #E01140 analytical balance, a Forma Model #F1214 biosafety cabinet (Marietta, Ohio), various pipettes to deliver 0.1 to 100 µL (VWR, Rochester, NY), a cell hand tally counter (VWR Catalog #23609-102, Rochester, NY), a Forma Model #F3210 CO2 incubator (Marietta, Ohio), a hemacytometer (Hausser Model #1492, Horsham, PA), a Leica Model #DM IL inverted microscope (Wetzlar, Germany), a PURELAB Plus Water Polishing System (U.S. Filter, Lowell, MA), a 4°C refrigerator (Forma Model #F3775, Marietta, Ohio), a vortex mixer (VWR Catalog #33994-306, Rochester, NY), and a 37°C water bath (Shel Lab Model #1203, Cornelius, OR).

*Cell culture -* RAW 264.7 cells, obtained from American Type Culture Collection (Catalog #TIB-71, Manassas, VA), were grown in Dulbecco's Modification of Eagle's Medium (DMEM, Mediatech, Herndon, VA) and maintained in log phase. The DMEM growth medium was made by adding 50 mL of heat inactivated FBS and 5 mL of penicillin/streptomycin to a 500 mL bottle of DMEM and storing at 4°C. The growth medium was warmed to 37 °C in water bath before use.

On day one of the experiment, the log phase RAW 264.7 cells were plated at 8 x 10⁴ cells per well in 0.2 mL growth medium per well in a 96-well tissue culture plate in the morning. At the end of the day one (6 to 8 h post plating), 100 µL of growth medium from each well were removed and replaced with 100 µL fresh medium.

A 1.0 mg/mL stock solution of LPS, used to induce the expression of COX-2 in the RAW 264.7 cells, was prepared by dissolving 1.0 mg of LPS in 1 mL DMSO. It was vortexed until dissolved and stored at 4°C. Before use, it was melted at room temperature or in a 37°C water bath.

On day two of the experiment, test materials were prepared as 1000X stock in DMSO. In 1.7 mL microfuge tubes, 1 mL DMEM without FBS was added for test concentrations of 0.05, 0.10, 0.5, and 1.0 µg/mL. Two µL of the 1000X DMSO stock of the test material was added to the 1 mL of medium without FBS. The tube contained the final concentration of the test material concentrated 2-fold and was placed in an incubator for 10 minutes to equilibrate to 37°C.

For COX-2 associated PGE₂ synthesis, 100 µL of medium were removed from each well of the cell plates prepared on day one and replaced with 100 µL of equilibrated 2X final concentration of the test compounds. Cells were then incubated for 90 minutes. Twenty µL of LPS were added to each well of cells to be stimulated to achieve a final concentration of 10 ng LPS/mL and the cells were incubated for 4 h. Following the LPS stimulation, the appearance of the cells was observed, and cell viability was assessed by a 3-(4,5-dimethylthiazol-2-yl)-2,5-diphenyltetrazolium bromide (MTT)-based colorimetric assay (Sigma, St. Louis, MO). The MTT solution was added directly to the wells after sampling for PGE₂ determination. The absorbance of each well was read at 580 nm using an ELISA plate reader. No toxicity was observed at the highest concentrations tested for any of the compounds.. No toxicity was observed at the highest concentrations tested for any of the compounds. Twenty-five µL of supernatant medium from each well was transferred to a clean microfuge tube for the determination of PGE2 released into the medium.

PGE₂ was assayed using a commercial, non-radioactive procedure for quantification of PGE₂ (Caymen Chemical, Ann Arbor, MI), and the recommended procedure of the manufacturer was used without modification. Briefly, 25 µL of the medium, along with a serial dilution of PGE₂ standard samples, were mixed with appropriate amounts of acetylcholinesterase-labeled tracer and PGE₂ antiserum, and incubated at room temperature for 18 h. After the wells were emptied and rinsed with wash buffer, 200 µL of Ellman's reagent containing substrate for acetylcholinesterase were added. The reaction was carried out on a slow shaker at room temperature for 1 h and the absorbance at 415 nm was determined. The PGE₂ concentration was represented as picograms per 10⁵ cells.

For COX-1 associated PGE₂ synthesis, 100 µL of medium were removed from each well of the cell plates prepared on day one and replaced with 100 µL of equilibrated 2X final concentration of the test compounds. Cells were then incubated for 90 minutes. Next, instead of LPS stimulation, the cells were incubated with 100 µM arachidonic acid for 15 minutes. Twenty-five µL of supernatant medium from each well was transferred to a clean microfuge tube for the determination of PGE₂ released into the medium. The appearance of the cells was observed and viability was determined as described above. No toxicity was observed at the highest concentrations tested for any of the compounds. Twenty-five µL of supernatant medium from each well was transferred to a clean microfuge tube for the determination of PGE₂ released into the medium. PGE₂ was determined and reported as described above. The median inhibitory concentrations (IC₅₀) for PGE₂ synthesis from both COX-2 and COX-1 were calculated as described below.

The median inhibitory concentration (IC₅₀) for PGE₂ synthesis was calculated using CalcuSyn (BIOSOFT, Ferguson, MO). This statistical package performs multiple drug dose-effect calculations using the median effect methods described by Chou and Talaly, Adv. Enzyme Regul. 22:27-55. (1984), hereby incorporated by reference.

Briefly, the analysis correlates the "Dose" and the "Effect" in the simplest possible form: fa/fu = (C/Cm)m, where C is the concentration or dose of the compound and Cm is the median-effective dose signifying the potency. Cm is determined from the x-intercept of the median-effect plot. The fraction affected by the concentration of the test material is fa and the fraction unaffected by the concentration is fu (fu = 1 - fa). The exponent m is the parameter signifying the sigmoidicity or shape of the dose-effect curve, It is estimated by the slope of the median-effect plot.

The median-effect plot is a graph of x= log(C) vs y = log(fa/fu) and is based on the logarithmic form of Chou's median-effect equation. The goodness of fit for the data to the median-effect equation is represented by the linear correlation coefficient r of the median-effect plot. Usually, the experimental data from enzyme or receptor systems have an r > 0.96, from tissue culture an r > 0.90 and from animal systems an r > 0.85. In the cell-based studies reported here, all linear correlation coefficients were greater than 0.90. Experiments were repeated three times on three different dates. The percent inhibition at each dose was averaged over the three independent experiments and used to calculate the median inhibitory concentrations reported.

**Table 3. COX-2 and COX-1 inhibition in RAW 264.7 cells by hop fractions and derviatives**

| Test Material | | COX-2 IG₅₀ [µg/mL] | COX-1 IC₅₀ [µg/mL] | COX-1 IC₅₀/COX-2 IC₅₀ |
|---|---|---|---|---|
| **Genus A structures** | | | | |
| | Isohop (IAA) | 0.13 | 18 | 144 |
| | Redihop (RIAA) | 0.34 | 29 | 87 |
| **Genus B structures** | | | | |
| | Tetrahop (THIAA) | 0.20 | 4.0 | 21 |
| | Hexahop (HHIAA) | 0.29 | 3.0 | 11 |
| **Alpha acids** | | | | |
| | Alpha hop (AA) | 0.21 | 6.2 | 30 |
| **Others** | | | | |
| | Aromahop OE | 1.6 | 4.1 | 2.6 |
| | Beta acids (BA) | 0.54 | 29 | 54 |
| | Spent hops (EtOH) | 0.88 | 21 | 24 |

As seen in Table 3, all hops fractions and derivatives selectively inhibited COX-2 over COX-1 in this target macrophage model. This was a novel and unexpected finding. The extent of COX-2 selectivity for the hops derivatives IAA and RIAA, respectively, 144- and 87-fold, was unanticipated. In this RAW 264.7 cell model, Genus A compounds exhibited a greater COX-2 selectivity than Genus B compounds, averaging 116-fold vs 16-fold, respectively, greater COX-2 inhibition. Alpha acid, beta acids and spent hops were also highly selective COX-2 inhibitors with COX-1/COX-2 ratios, respectively, 30, 54 and 24. Such high COX-2 selectivity combined with low median inhibitory concentrations, has not been previously reported for natural products from other sources. Aromahop was least COX-2 selective with a COX-1/COX-2 ratio of 2.6.

### EXAMPLE 2

### LACK OF INHIBITION OF PGE₂ SYNTHRSIS IN GASTRIC MUCOSAL CELLS BY HOPS (Humulus lupulus) COMPOUNDS AND DERVIATIVES

Summary - This example illustrates the lack of PGE₂ inhibition by hops fractions and in the AGS human gastric mucosal cell line implying low gastric irritancy potential of these compounds.

Chemicals and reagents were used as described in EXAMPLE 1. PGE₂ was determined and reported as previously described in EXAMPLE 1. The median inhibitory concentrations (IC₅₀) for PGE₂ synthesis from AGS cells were calculated as described in EXAMPLE 1[2].

The human gastric mucosal cell line AGS was obtained from the American Type Culture Collection (ATCC number CRL-1739; Manassas, VA) and sub-cultured according to the instructions of the supplier. The cells were routinely cultured at 37°C with 5% CO₂ in RPMI 1640 containing 10% FBS, with 50 units penicillin/mL, 50 µg streptomycin/mL, 5% sodium pyruvate, and 5% L-glutamine. Exponentially growing cells were seeded into 6-well plates and grown to confluence. A 20 µL aliquot of the supernatant media was sampled for determination of PGE₂ content. Cells were then washed in PBS, scraped and lysed for immunoblotting.

**Table 4. Median inhibitory concentrations (IC₅₀) for PGE₂ synthesis of hops derivatives in the AGS cell model†**

| Compound | | AGS IC₅₀ [µg/mL] | 95% Confidence Interval [µg/mL] | r |
|---|---|---|---|---|
| | | | | |
| **Alpha Acids** | | | | |
| | Alphahop | 17 | 2.4 - 124 | 0.927 |
| | | | | |
| **Genus A structures**†† | | | | |
| **Iso-alpha acids** | | | | |
| | Isohop (IAA) | 16 | 4.9 - 54 | 0.970 |
| | Isorich | 9.2 | 1.1 - 81 | 0.894 |
| **Reduced iso-alpha acids** | | | | |
| | Redihop (RIAA) | 21 | 3.1 - 145 | 0.936 |
| | | | | |
| **Genus B structures**††† | | | | |
| **Tetra-iso-alpha acids** | | | | |
| | Tetrahop (THIAA) | 51 | 6.8 - 374 | 0.949 |
| **Hexa-iso-alpha acids** | | | | |
| | Hexahop (HHIAA) | 34 | 25 - 47 | 0.998 |
| | | | | |
| **Others** | | | | |
| | BetaStab | 73 | 18 - 291 | 0.977 |
| | Tannin extract #4411 | 59 | 11 - 324 | 0.963 |
| | Aromahop | 43 | 21 - 85 | 0.992 |
| | #1115 (Spent hops) | 35 | 8.5 - 141 | 0.970 |
| | | | | |
| **Positive Concurrent Control** | | | | |
| | Aspirin | 1.2 | (0.37 - 4.1) | 0.950 |
| **Historical Control** | | | | |
| | Aspirin | 0.52 | (0.26 *-* 1.0) | - |
| | Ibuprofen | 0.57 | (0.27 - 1.2) | - |
| | Rofecoxib | 1.8 | (0.90 - 3.7) | - |
| | Celcoxib | 0.024 | (0.0068 - 0.082) | - |

| | | | | |
|---|---|---|---|---|
| † IC₅₀ values are computed from the average of three independent assays; AGS cells were plated and allowed to reach 80% confluence. Cells were washed and test material was added 60 minutes prior to treatment with A23187. Thirty minutes later, media was removed for PGE₂ determination. | | | | |

Median inhibitory concentrations for PGE₂ synthesis of hops derivatives in the AGS cell model are presented in Table 4. Genus B structures, in general, were less inhibitory than Genus A structures and alpha acids. In the Genus B group, IC₅₀ values for THIAA and HHIAA were, respectively, 51 and 34 µg/mL. IC₅₀ values for IAA, Isorich and RIAA from the Genus A group were, respectively, 16, 9.2 and 21 µg/mL, on average 63% lower than IC₅₀ values from Genus B species. With relatively high IC₅₀ values, hops derivatives BetaStab (73 µg/mL), Tannin extract #4411 (59 µg/mL), Aromahop (43 µg/mL) and #1115 spent hops (35 µg/mL) would rank as non-irritating to the gastric mucosa. Unexpectedly, all hops derivatives were substantially less inhibitory to AGS gastric mucosal cells than any NSAID including the newer, highly selective COX-2 drugs rofecoxib and celecoxib.

### EXAMPLES 3

### ACUTE TOXICITY OF GENUS A AND GENUS B HOPS DERIVATES IN RATS

The acute toxicity of hops derivatives is examined in rats. Ten, young, Fisher 344 male rats averaging 100 g are orally dosed with 5000 mg test material/kg body weight and observed for 14 days; the number of dead rats is determined. The low acute toxicity of hops derivatives is illustrated by lack of lethality when administered to rats orally at 5,000 mg test material/kg body weight.

### EXAMPLE 4

### ASSESSING SYNERGY OF PGE2 INHIBITION PRODUCED BY COMBINATIONS OF REDUCED ISOMERIZED ALPHA ACIDS AND ISOMERIZED ALPHA ACIDS IN RAW 264.7 CELLS

This example describes the effect of combinations of reduced isomerized alpha acids (RIAA) and isomerized alpha acids (IAA) on the inhibition of prostaglandin E₂ (PGE₂) production in the lipopolysaccharide (LPS)-stimulated RAW 264.7 model of inflammation.

The standard equipment used in these experiments is described in Example 1. Chemicals and reagents were obtained as follows. Bacterial lipopolysaccharide (LPS; B E. coli 055:B5) was from Sigma (St. Louis, MO). Prostaglandin E₂ monoclonal antibody kit was purchased from Cayman Chemical (Ann Arbor, MI). Heat inactivated Fetal Bovine Serum (FBS-HI Cat. #35-011CV) and Dulbecco's Modification of Eagle's Medium (DMEM Cat #10-1013CV) was purchased from Mediatech (Herndon, VA). Unless otherwise noted, all standard reagents were obtained from Sigma (St. Louis, MO) and were the purest commercially available. Test substances included RIAA (Redihop (rho-iso-alpha acids (RIAA), 29.5 - 30.5%, <0.2% iso-alpha acids)) and IAA (Isohop (iso-alpha acids (IAA), 29.5 - 30.5%) obtained from Betatech Hops Products (Washington, DC).

*Cell culture and treatment with test material -* RAW 264.7 cells (ATCC number TIB-71) were obtained from the American Type Culture Collection (Manassas, VA) and sub-cultured according to the instructions of the supplier. In preparation for testing, cells were grown in growth DMEM medium with 10% FBS-HI with penicillin/streptomycin and maintained in log phase prior to experimental setup. On day two of the experiment, cells were plated at 8 x 10⁴ cells per well in a 96-well tissue culture plate with 200 µL growth medium per well.

Following overnight incubation at 37°C with 5% CO₂, the growth medium was aspirated and replaced with 200 µL DMEM with no fetal bovine serum (FBS) or penicillin/streptomycin. Test materials were dissolved in dimethylsulfoxid (DMSO) as a 250-fold stock solution. Four µL of this 250-fold stock test material preparation was added to 1 mL of DMEM and 200 µL of this solution was added to wells in duplicate for each dose of test material. Final concentrations of test material were 10, 1, 0.1 and 0.01 µg/mL. Table 5 describes the dosing matrix for RAW 264.7 cells treated with test material and LPS stimulation.

**Table 5. Dosing matrix for RAW 264.7 cells treated with test material and LPS stimulation.**

| Compound | Fraction RIAA | | d1 [µg/mL] | d2 [µg/mL] | d3 [µg/mL] | d4 [µg/mL] | No. Wells |
|---|---|---|---|---|---|---|---|
| 1. BetaTech RIAA | 1.00 | | 10.000 | 1.000 | 0.100 | 0.010 | 8 |
| 2. BetaTech IAA | 0.00 | | 10.000 | 1.000 | 0.100 | 0.010 | 8 |
| | | | | | | | |
| 3. RIAA:IAA [100:1] | 0.99 | | 10.000 | 1.000 | 0.100 | 0.010 | 8 |
| | | RIAA = | 9.901 | 0.990 | 0.099 | 0.0099 | |
| | | IAA = | 0.099 | 0.010 | 0.001 | 0.0001 | |
| 4. RIAA:IAA [10:1] | 0.91 | | 10.000 | 1.000 | 0.100 | 0.010 | 8 |
| | | RIAA = | 9.091 | 0.909 | 0.091 | 0.009 | |
| | | IAA = | 0.909 | 0.091 | 0.009 | 0.001 | |
| 5. RIAA:IAA [3:1] | 0.75 | | 10.000 | 1.000 | 0.100 | 0.010 | 8 |
| | | RIAA = | 7.500 | 0.750 | 0.075 | 0.0075 | |
| | | IAA = | 2.500 | 0.250 | 0.025 | 0.0025 | |
| 6. RIAA:IAA [3:2] | 0.60 | | 10.000 | 1.000 | 0.100 | 0.010 | 8 |
| | | RIAA = | 6.00 | 0.60 | 0.06 | 0.006 | |
| | | IAA = | 4.00 | 0.40 | 0.04 | 0.004 | |
| 7. RIAA:IAA [1:1] | 0.50 | | 10.000 | 1.000 | 0.100 | 0.010 | 8 |
| | | RIAA = | 5.00 | 0.50 | 0.05 | 0.005 | |
| | | IAA = | 5.00 | 0.50 | 0.05 | 0.005 | |
| 8. RIAA:IAA [2:3] | 0.40 | | 10.000 | 1.000 | 0.100 | 0.010 | 8 |
| | | RIAA = | 4.00 | 0.40 | 0.04 | 0.00 | |
| | | IAA = | 6.00 | 0.60 | 0.06 | 0.01 | |
| 9. RIAA:IAA [1:10] | 0.09 | | 10.000 | 1.000 | 0.100 | 0.010 | 8 |
| | | RIAA = | 0.909 | 0.091 | 0.009 | 0.001 | |
| | | IAA = | 9.091 | 0.909 | 0.091 | 0.009 | |
| 10. RIAA:IAA [1:100] | 0.010 | | 10.000 | 1.000 | 0.100 | 0.010 | 8 |
| | | RIAA = | 0.10 | 0.01 | 0.001 | 0.0001 | |
| | | IAA = | 9.90 | 0.99 | 0.099 | 0.0099 | |

*Determination of PGE₂ -* A commercial, non-radioactive procedure for quantification of PGE₂ was employed (Caymen Chemical, Ann Arbor, MI) for the determination of PGE₂, and the recommended procedure of the manufacturer was used without modification. In summary, 50 µL of the supernatant culture medium were diluted with appropriate amounts of acetylcholinesterase-labeled tracer and PGE₂ antiserum and incubated at room temperature for 18 h. Afterwards, the wells in the PGE₂-assay microtiter plate were emptied and rinsed with wash buffer; two-hundred µL of Ellman's reagent containing substrate for acetylcholinesterase were then added. The reaction was maintained on a slow shaker at room temperature for 1 h and the absorbance at 415 nm was determined in a Bio-tek Instruments (Model #E1x800, Winooski, VT) ELISA plate reader. The manufacturer's specifications for this assay include an intra-assay coefficient of variation of <10%, cross reactivity with PGD2 and PGF_{2α} of less than 1% and linearity over the range of 10 - 1000 pg mL-1. The PGE₂ concentration was computed as pg PGE₂ per 10⁵ cells, as described below.

For calculations related to PGE₂ assays, the median inhibitory concentration (IC₅₀) for PGE₂ synthesis was calculated using CalcuSyn (BIOSOFT, Ferguson, MO). This statistical package performs multiple drug dose-effect calculations using the median effect methods described by Chou and Talaly (Adv. Enzyme Regul. 22:27-55 (1984)).

Briefly, the analysis correlates the "Dose" and the "Effect" in the simplest possible form: fa/fu = (C/Cm)^{m}, where C is the concentration or dose of the compound and Cm is the median-effective dose signifying the potency. Cm is determined from the x-intercept of the median-effect plot. The fraction affected by the concentration of the test material is fa and the fraction unaffected by the concentration is fu (fu = 1 - fa). The exponent m is the parameter signifying the sigmoidicity or shape of the dose-effect curve. It is estimated by the slope of the median-effect plot

The median-effect plot is a graph of x= log(C) vs y = log(fa/fu) and is based on the logarithmic form of Chou's median-effect equation. The goodness of fit for the data to the median-effect equation is represented by the linear correlation coefficient r of the median-effect plot. Usually, the experimental data from enzyme or receptor systems have an r > 0.96, from tissue culture an r > 0.90 and from animal systems an r > 0.85. In the cell-based studies reported here, all linear correlation coefficients were greater than 0.90. For most robust results, experiments are repeated a minimum of three times on three different dates. The percent inhibition at each dose is averaged over the three independent experiments and used to calculate the median inhibitory concentrations reported.

Synergy of test components is quantified using the combination index (CI) parameter. The CI of Chou-Talaly is based on the multiple drug-effect and is derived from enzyme kinetic models (Chou and Talaly, J. Biol. Chem. 252:6438-6442 (1977)). The equation determines only the additive effect rather than synergism or antagonism. However, synergism is defined in this analysis as a more than expected additive effect, and antagonism as a less than expected additive effect as proposed by Cho and Talaly. Using the designation of CI = 1 as the additive effect, for mutually exclusive compounds that have the same mode of action or for mutually non-exclusive drugs that have totally independent modes of action, the following relationships are obtained: CI < 1, = 1, and > 1 indicating synergism, additivity and antagonism, respectively.

*Cell viability* - Cell viability was assessed by visual inspection of cells prior to or immediately following sampling of the medium for PGE2 assay. Cell mortality was noted when observed.

For statistical methods, a minimum of four concentrations (Table 5) was used to compute dose-response curves and medium inhibitory concentrations (IC₅₀s) with 95% confidence intervals using CalcuSyn (BIOSOFT, Ferguson, MO). This statistical package performs multiple drug dose-effect calculations using the Median Effect methods described by Chou and Talaly, *supra*, 1984. All dose-response data captured the median inhibitory concentration (Appendix B). Two data transformations were applied where warranted. The first transformation consisted of computing the percent inhibition from the highest PGE₂ production produced from the lowest test concentration when the PGE₂ production of these low doses exceeded the PGE₂ production of the LPS-stimulated control. This process controls for response variability and gradients throughout the plate. The second data transformation adjusted for variance in response at the graded doses. Monte Carlo simulations using the historical variance between wells predicted that dose-response curves appear graded only 40% of the time when duplicate wells per concentration are used in a four-point dose-response curve. Thus, sorting the response by concentration before calculating the IC₅₀ was done in those situations in which the response did not appear graded.

Results - RIAA and IAA median inhibitory concentration (IC₅₀) values of 0.24 µg/mL (95% confidence limit (CL) 0.060 - 0.94 µg/mL) and 0.56 µg/mL (95% CL 0.28 - 1.1), respectively, obtained in this study were consistent with previous results in this laboratory using the LPS-RAW 264.7 overnight protocol.

Median inhibitory concentrations for RIAA, RIAA and RIAA:IAA combinations for LPS-stimulated RAW 264.7 cells are presented in Table ,6 with the regions of synergy computed for each combination. Surprisingly, synergy was noted for all RIAA:IAA combinations, albeit at different segments of the dose-response curves. Regions of synergy were seen at the lower portion of the dose-response curves for RIAA:IAA combinations of 10:1, 1:1 and 1:100, covering RIAA concentrations of 2.5x10⁻⁸ to 0.26 µg/mL. Synergy was noted at the higher end of the dose-response curve for RIAA:IAA, ratios of 100:1, 3:1, 3:2, 2:3 and 1:10 over RIAA concentrations of 0.31 to 68,261 µg/mL. Thus, it is reasonable to expect synergy to occur in vivo over a wide range of doses of both RIAA and IAA regardless of the ratio of the components in the formulation dosed.

**Table 6. Median inhibitory concentrations and regions of synergy for RIAA, IAA and RIAA:IAA combinations in LPS-stimulated RAW264.7 cells.**

| Test Material | RIAA [%] | IC₅₀ [µg/mL] | Region of Synergy RIAA [µg/mL] |
|---|---|---|---|
| | | | |
| RIAA | 100 | 0.24 | |
| IAA | 0 | 0.56 | |
| RIAA:IAA [100:1] | 99 | 0.68 | 955 - 55655 |
| RIAA:IAA [10:1] | 91 | 0.28 | 1.1x10⁻⁶ - 0.070 |
| RIAA:IAA [3:1] | 75 | 1.8 | 8.2 - 531 |
| RIAA:IAA [3:2] | 60 | 1.1 | 10-5807 |
| RIAA:IAA [1:1] | 50 | 0.23 | 2.4x10⁻⁸ - 0.26 |
| RIAA:IAA [2:3] | 40 | 1.9 | >1127 |
| RIAA:IAA [1:10] | 9.1 | 0.77 | 0.31 - 4661 |
| RIAA:IAA [1:100] | 1.0 | 0.60 | 1.5x10⁻⁸ - 6.0x10⁻⁵ |

| | | | |
|---|---|---|---|
| Region of synergy defined by CI<1.0 | | | |

RAW 264.7 cells were treated with test material 60 minutes prior to LPS stimulation and incubated overnight. Eighteen hours post LPS-stimulation, supernatant media was sampled for PGE₂ determination. Median inhibitory concentrations were computed from a minimum of four concentrations over two independent experiments. The CIs were computed as described above.

Figure 4 shows a graphic representation of the computed Combination Index parameter versus the concentration of reduced isomerized alpha-acids (RIAA), isomerized alpha-acids (IAA), and for RIAA:IAA ratios of 100:1 (Figure 4A), 10:1 (Figure 4B), 3:1 (Figure 4C), 3:2, (Figure 4D), 1:1 (Figure 4E), 2:3 (Figure 4F), 1:10 (Figure 4G), 1:100 (Figure 4H).

These results show that synergy between RIAA and IAA was observed in four combinations - 3:1, 3:2, 1:1 and 1:10. Particularly relevant synergy occurred at the 1:1 and 1:10 RIAA:IAA ratios. For these formulations, synergy was seen, respectively, at RIAA concentrations < 0.58 µg/mL and RIAA concentrations > 0.31 µg/mL.

Throughout this application various publications have been referenced. The disclosures of these publications in their entireties are hereby incorporated by reference in this application in order to more fully describe the state of the art to which this invention pertains. Although the invention has been described with reference to the examples provided above, it should be understood that various modifications can be made without departing from the spirit of the invention.
The present invention also relates to the following items:
1. A composition comprising a reduced isoalpha acid (RIAA) and isoalpha acid (IAA) isolated from hops, wherein the RIAA and IAA are in a ratio of about 3:1 to about 1:10 and wherein said RIAA and IAA individually comprise at least 0.1% of the composition.
2. The composition of item 1, wherein said isoalpha acid is selected from isohumulone, isocohumulone, and isoadhumulone.
3. The composition of item 1, wherein said reduced isoalpha acid is selected-from dihydro-isohumulone, dihydro-isocohumulone, and dihydro-adhumulone.
4. A method for reducing PGE2 mediated inflammation, comprising administering a composition comprising a reduced isoalpha acid (RIAA) and isoalpha acid (IAA) isolated from hops, wherein the RIAA and IAA are in a ratio of about 3:1 to about 1:10 and wherein said RIAA and IAA individually comprise at least 0.1% of the composition.
5. The method of item 4, wherein said isoalpha acid is selected from isohumulone, isocohumulone, and isoadhumulone.
6. The method of item 4, wherein said reduced isoalpha acid is selected from dihydro-isohumulone, dihydro-isocohumulone, and dihydro-adhumulone.
7. A method FOR reducing PGE2 mediated inflammation, comprising administering at least two compounds of Genus A having the formula: 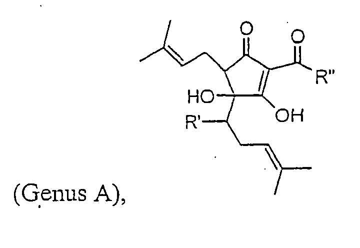 wherein R' is selected from the group consisting of carbonyl, hydroxyl, OR, and OCOR, wherein R is alkyl;
   and wherein R" is selected from the group consisting of CH(CH₃)₂, CH₂CH(CH₃)₂, and CH(CH₃)CH₂CH₃, wherein the two compounds are in a ratio of about 10:1 to about 1:10 and wherein said RIAA and IAA individually comprise at least 0.1 % of the composition.

## Claims

1. A composition comprising a reduced isoalpha acid (RIAA) and an isoalpha acid (IAA), wherein the RIAA and IAA are in a ratio of about 3:1 to about 1:10; said RIAA and IAA individually comprise at least 0.1 % of the composition; and the combination of said RIAA and IAA in said composition provides a combination index (CI) of less than 1 in relation to the inhibition of PGE2 production; for use in reducing PGE2-mediated inflammation.

2. The composition for use according to claim 1, wherein the RIAA and IAA are derived from hops.

3. The composition for use according to claim 1, wherein the RIAA is selected from the group consisting of dihydro-isohumulone, dihydro-isocohumulone and dihydro-isoadhumulone.

4. The composition for use according to claim 1, wherein the IAA is selected from the group consisting of isohumulone, isocohumulone and isoadhumulone.

5. The composition for use according to claim 1, wherein the composition comprises from about 50 mg to about 7500 mg of the RIAA.

6. The composition for use according to claim 1, wherein the composition comprises from about 50 mg to about 7500 mg of the IAA.

7. The composition for use according to claim 1, wherein the composition further comprises a pharmaceutically acceptable carrier.

8. The composition for use according to claim 1, wherein the composition is administered orally, topically, parenterally, or rectally.
